# EUROPEAN PATENT APPLICATION

(11) **EP 2 394 699 A2**
(43) Date of publication of application: **14.12.2011**
(21) Application number: 11168210.0
(22) Date of filing: 31.05.2011
(51) Int. Cl.: A61N 2/08

(54) **Blood oxidation inhibiting apparatus**

(30) Priority: 11.06.2010 JP 2010134233
(71) Applicant: Japan System Planning Co., Ltd., Tokyo 151-0073 (JP)
(72) Inventor: Kumano, Katsuyuki, Tokyo Tokyo 151-0073 (JP); Yamamoto, Naomasa, Saitama-ken Saitama 330-0842 (JP); Yonehara, Norifumi, Fukushima-ken Fukushima 963-8041 (JP); Koike, Yuichi, Fukushima-ken Fukushima 963-8026 (JP)
(74) Representative: Harrison, Michael Robert

(57) **Abstract**

[Problem to be Solved] A compact and light weight, and inexpensive blood oxidation inhibiting apparatus which uses fluid activating bodies, being capable of inhibiting oxidative stress on the blood of a human body is provided.

[Solution] A blood oxidation inhibiting apparatus, a finger insertion hole 1 for inserting a finger being formed therein, comprising a plurality of fluid activating bodies 2A, 2B, 2C being disposed around the finger insertion hole 1, the fluid activating bodies 2A, 2B, 2C being integrally fixed to a cover 10 with an epoxy resin 5, the cover 10 being formed of straight line portions 11, 12 extending substantially at right angles to each other and a circular arc portion 13 connecting between the ends of both straight line portions 11, 12 in the shape of a circular arc, and the fluid activating bodies 2A, 2B, 2C being constituted by a set of at least three fluid activating bodies, two 2A, 2B thereof being disposed between one of both ends of the circular arc portion 13 and the finger insertion hole 1, and between the other of both ends of the circular arc portion 13 and the finger insertion hole 1, respectively, with the electromagnetic wave converging body 4 thereof being oriented toward the finger insertion hole 1, and one 2C thereof being disposed between the corner formed by both straight line portions 11, 12 and the finger insertion hole 1 with the electromagnetic wave converging body 4 thereof being oriented toward the finger insertion hole 1.

## Description

### BACKGROUND OF THE INVENTION

### Field of the Invention

The present invention relates to a blood oxidation inhibiting apparatus.

### Description of the Related Art

Conventionally, as a fluid activation apparatus, that as disclosed in the patent document 1 is known. This apparatus includes fluid activating bodies disposed around the outside periphery of a pipe in order to activate a fluid flowing in the pipe. Each of the fluid activating bodies is composed of a black radiation sintered body made by sintering the powder of a plurality of kinds of metal oxides at high temperature and an electromagnetic wave converging body for converging the electromagnetic wave radiated from the black radiation sintered body to a specific wavelength. The electromagnetic wave converging body is formed by laminating at least six magnets with the N-poles and the S-poles thereof disposed alternately, and an electromagnetic wave passing-through hole is formed to the laminated magnets so as to pass therethrough.
Patent document 1: Japanese Patent No. 3952477 (Japanese Patent Laid-Open No. 2006-68621)

With the prior art as described above, the object thereof is merely to solve the problem of fluid activation, and thus the effect on the blood of a human body is not completely expected. The present inventors organized a collaborative team and carried out experiments for examining whether the above-described prior art can exert an effect on the blood of a human body as later described. As a result of this, it has been found that the above-described prior art, if used in conjunction with electromagnetic wave irradiation, can lower and eliminate the oxidative stress, exerting an effect on diseases attributed to the oxidative stress, and is applicable to therapy of insomnia, and the like.

It is an object of the present invention to provide a compact and light weight, and inexpensive blood oxidation inhibiting apparatus which uses fluid activating bodies, being capable of inhibiting oxidative stress on the blood of a human body.

### SUMMARY OF THE INVENTION

According to the invention as stated in claim 1, there can be provided a blood oxidation inhibiting apparatus, a finger insertion hole for inserting a finger being formed therein, comprising a plurality of fluid activating bodies being disposed around the finger insertion hole, the fluid activating bodies being integrally fixed to a cover with an epoxy resin, the cover being formed of straight line portions extending substantially at right angles to each other and a circular arc portion connecting between the ends of both straight line portions in the shape of a circular arc, and the fluid activating bodies being constituted by a set of at least three fluid activating bodies, two thereof being disposed between one of both ends of the circular arc portion and the finger insertion hole, and between the other both ends of the circular arc portion and the finger insertion hole, respectively, with the electromagnetic wave converging body thereof being oriented toward the finger insertion hole, and one thereof being disposed between the corner formed by both straight line portions and the finger insertion hole with the electromagnetic wave converging body thereof being oriented toward the finger insertion hole.

According to the invention as stated in claim 2, there can be provided a blood oxidation inhibiting apparatus, a finger insertion hole for inserting a finger being formed therein, comprising a plurality of fluid activating bodies being disposed around the finger insertion hole, with the electromagnetic wave converging body thereof being oriented toward the finger insertion hole, the fluid activating bodies being integrally fixed to a cover with an epoxy resin, the cover being formed substantially in the shape of a square, and the fluid activating bodies being constituted by a set of at least four fluid activating bodies each disposed between one of the four corners of the cover and the finger insertion hole with the electromagnetic wave converging body thereof being oriented toward the finger insertion hole.

When a person inserts a thumb or an index finger thereof into the insertion hole, the blood vessels in the finger are irradiated with a specific laser-like electromagnetic wave from any of the three or four fluid activating bodies. Thereby, the oxidative stress in the blood is lowered and eliminated, which exerts an effect on the diseases attributed to the oxidative stress, thereby insomnia, and the like, being treated. Further, the blood oxidation inhibiting apparatus of the present invention is of finger insertion type, and has a simple construction containing fluid activating bodies, thus it is compact and light weight, and can be manufactured inexpensively.

### BRIEF DESCRIPTION OF THE DRAWINGS

Fig. 1 is a front view illustrating one embodiment of a blood oxidation inhibiting apparatus according to the present invention;
Fig. 2 is a plan view for Fig. 1;
Fig. 3 is a sectional view taken along the line A-A in Fig. 1;
Fig. 4 is a front view illustrating another embodiment of a blood oxidation inhibiting apparatus according to the present invention;
Fig. 5 is a graph illustrating the change in BAP/dROM ratio before and after the irradiation with the Yubi-MR on a healthy subject donor;
Fig. 6 is a graph illustrating the effect of the Yubi-MR for animal use on the BAP/dROM for mice;
Fig. 7 is a graph illustrating the relationship between the time period of irradiation and the motor activity for the Yubi-MR for animal use and the dummy apparatus;
Fig. 8 is a graph illustrating the comparison of the integrated value of the mouse motor activity on the irradiation with the Yubi-MR for animal use with that with the dummy apparatus;
Fig. 9 is a graph illustrating the recovery by the irradiation onto the mice with the dummy apparatus after the irradiation with the Yubi-MR for animal use;
Fig. 10 is a graph illustrating the recovery from the reduction in motor activity on the irradiation with the Yubi-MR for animal use;
Fig. 11 is a graph illustrating the frequency change in a time series on the irradiation with the magnet;
Fig. 12 is a graph illustrating the frequency change in a time series on the irradiation with the Yubi-MR for animal use;
Fig. 13 is a graph illustrating the periodgram on the irradiation with the magnet; and
Fig. 14 is a graph illustrating the periodgram on the irradiation with the Yubi-MR for animal use.

### DETAILED DESCRIPTION OF THE PREFERRED EMBODIMENTS

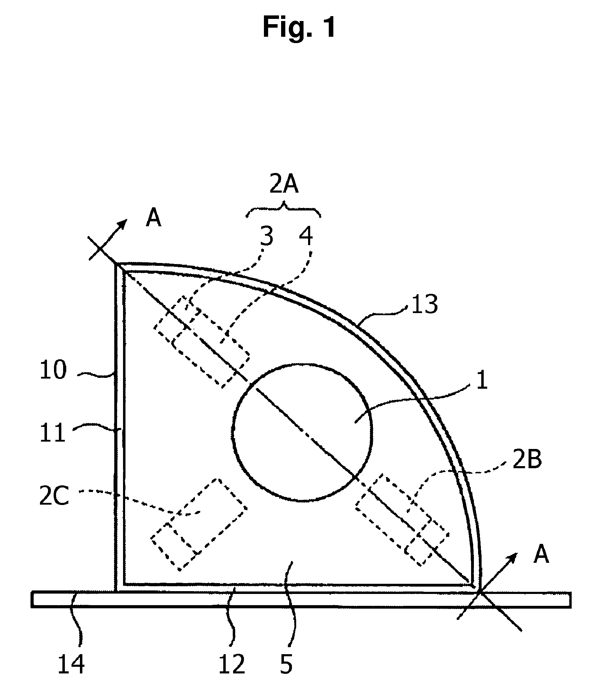

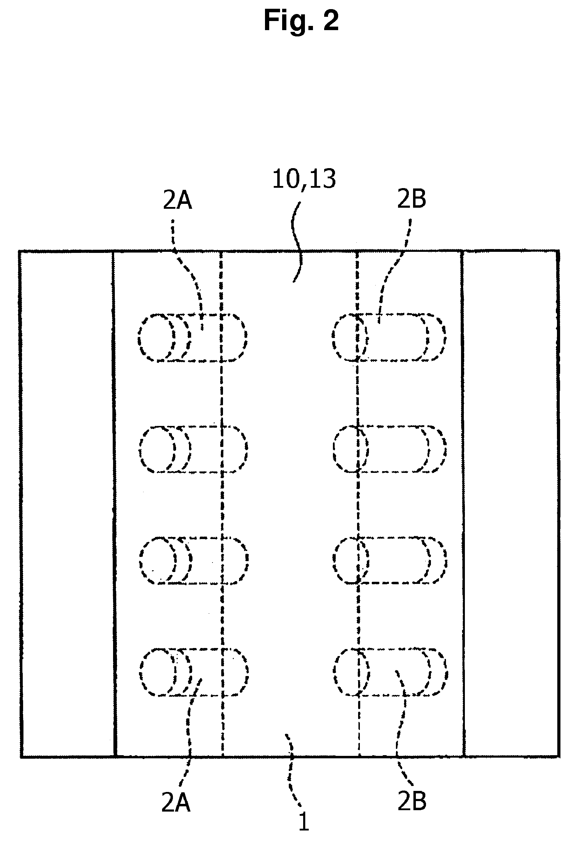

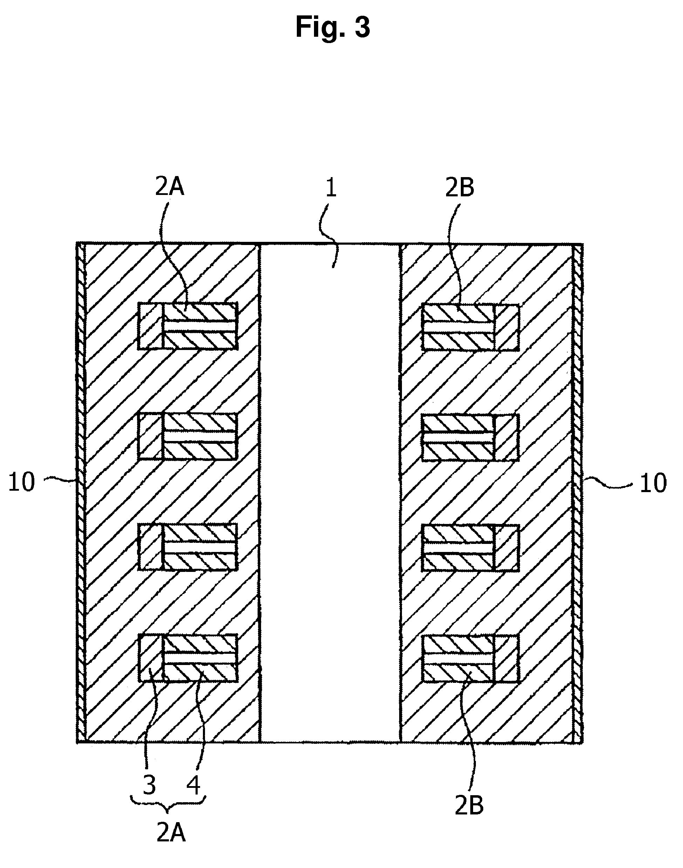

With reference to Fig. 1 to Fig. 3, one embodiment of a blood oxidation inhibiting apparatus according to the present invention will be described. The blood oxidation inhibiting apparatus is adapted to be of finger insertion type such that it is compact and light weight, and can be inexpensively manufactured, and used in home.

Around a finger insertion hole 1 for inserting a finger, four sets of three fluid activating bodies 2A, 2B, 2C are disposed. The fluid activating bodies 2A, 2B, 2C are each constituted as stated in the patent document 1. In other words, the fluid activating bodies 2A, 2B, 2C are each constituted by a black radiation sintered body 3 and an electromagnetic wave converging body 4 for converging the electromagnetic wave generated by this black radiation sintered body 3 to a specific wavelength. And the fluid activating bodies 2A, 2B, 2C are each disposed such that the electromagnetic wave converging body 4 is oriented toward the finger insertion hole 1.

The fluid activating bodies 2A, 2B, 2C are integrally fixed to a cover 10 with an epoxy resin 5. The cover 10 is formed of straight line portions 11, 12 extending substantially at right angles to each other and a circular arc portion 13 connecting between the ends of both straight line portions 11, 12 in the shape of a circular arc, the straight line portion 12 is fixed to a supporting plate 14. The supporting plate 14 need not always be provided. The finger insertion hole 1 is formed in the central portion on the line connecting between both ends of the circular arc portion 13. The fluid activating bodies 2A, 2B are disposed on the line connecting between the ends of the circular arc portion 13, being oriented toward the finger insertion hole 1, and the fluid activating body 2C is disposed between the corner formed by the straight line portions 11, 12 and the finger insertion hole 1, being oriented toward the finger insertion hole 1.

Next the function of the blood oxidation inhibiting apparatus will be described. A thumb or an index finger is inserted into the finger insertion hole 1. Because the three fluid activating bodies 2A, 2B, 2C are disposed substantially at intervals of 90 degrees, the blood vessels in the finger are irradiated with the electromagnetic wave from any of the fluid activating bodies 2A, 2B, 2C. Thereby, the oxidative stress in the blood is lowered and eliminated, which exerts an effect on diseases attributed to the oxidative stress, thereby insomnia, and the like, being treated. Further, the blood oxidation inhibiting apparatus of the present invention is of finger insertion type, and has a simple construction containing the fluid activating bodies 2A, 2B, 2C, thus it is compact and light weight, and can be manufactured inexpensively.

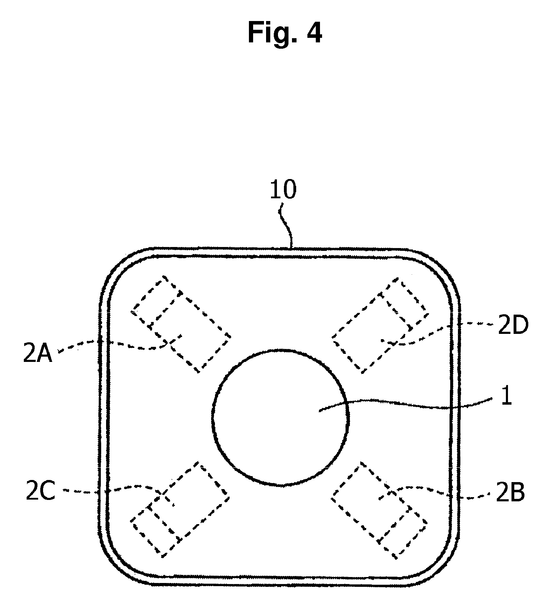

Fig. 4 illustrates another embodiment of a blood oxidation inhibiting apparatus according to the present invention. The same or equivalent components as those in the above-described embodiment will be provided with the same symbols in the following explanation. In the present embodiment, a set of four fluid activating bodies 2A, 2B, 2C, 2D are provided. The cover 10 is formed in the shape of a square, and the fluid activating bodies 2A, 2B, 2C, 2D are each disposed between one the four corners of the cover 10 and the finger insertion hole 1, being oriented from toward the finger insertion hole 1. Even if the blood oxidation inhibiting apparatus is configured in this way, the same effect as that described above can be obtained.

The above embodiments have been explained for an application where four sets of three or four fluid activating bodies are provided, however, the number of sets may be one or more, and not particularly defined.

Hereinbelow, experimental results demonstrating that the blood oxidation inhibiting apparatus of the present embodiment can eliminate the oxidative stress on a human and an animal, exerting an effect on diseases attributed to the oxidative stress, and is applicable to therapy to insomnia and the like will be described. In the following experiment, the "Yubi-MR" refers to a blood oxidation inhibiting apparatus of the present embodiment as shown in Fig. 1 to Fig. 3 in which a set of three fluid activating bodies 2A, 2B, 2C is provided. The "Yubi-MR for animal use" refers to a fluid activation apparatus which uses a "Pipetector" (the name of a product of Japan System Planning Co., Ltd.) and is configured as stated in claim 4 in the patent document 1 (Japanese Patent No. 3952477). The "dummy apparatus" refers to an apparatus loaded only with wiggler magnets, which was manufactured for comparison with the "Yubi-MR for animal use".

The "BAP" is an acronym of biological antioxidant potential. The "dROM" is an acronym of diacron reactive oxygen metabolites, meaning the oxidative stress. The BAP and dROM were measured, using FRAS4 (Free Radical Analytical System 4) manufactured by Wismerll Co., Ltd.

Experimenters: Naomasa Yamamoto is an Associate Professor in the School of Pharmaceutical Sciences at the Ohu University; Norifumi Yonehara a Professor in the School of Pharmaceutical Sciences at the Ohu University; and Yuuichi Koike a Professor in the School of Pharmaceutical Sciences at the Ohu University.
Experiment location: The laboratory in the Department of Biochemistry in the School of Pharmaceutical Sciences at the Ohu University

Experiment 1: Experiment using blood of human
Experimenters: Naomasa Yamamoto and Yuuichi Koike
Date of experiment: Nov. 10, 2009 to Feb. 7, 2010
Subjects: 9 healthy subjects (20 to 58 years old, four men, five women); BAP and dROM therefor were measured.
Method of blood collection and that of Yubi-MR irradiation: Using a winged needle 21G, 10 µL (1000 units/mL) of heparin was contained in a syringe (2.5 m), and 1.5 mL of blood was collected from an elbow vein for use as the blood before treatment. The Yubi-MR irradiation was carried out by keeping the winged needle loaded in the elbow vein and inserting the index finger of an arm on the same side into the finger insertion hole in the Yubi-MR, which was followed by keeping the subject in its resting state for 10 minutes. Thereafter, 1.5 mL of blood was drawn, which was followed by heparin-collecting another 1.5 mL of blood in the same manner, and the blood was used as the after-irradiation blood. Since this measurement was an experiment before filing a patent application for the Yubi-MR, the measurement subjects were told to keep the measurement confidential.

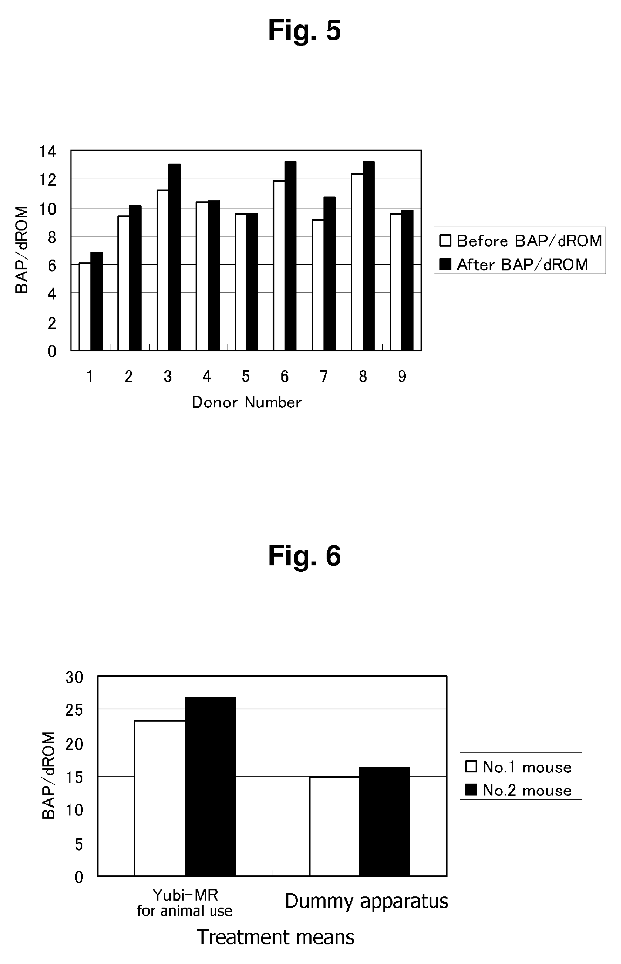

Results: Table 1 gives the changes in BAP and dROM before and after Yubi-MR irradiation for 9 subjects, and Fig. 5 shows the changes in BAP/dROM ratio before and after Yubi-MR irradiation for 9 subjects. As can be seen from Table 1 and Fig. 5, the BAP value was not significantly changed before and after the Yubi-MR irradiation, while the dROM value was significantly reduced (at a critical rate of 5% or less) before and after the Yubi-MR irradiation.
Consideration: The Yubi-MR irradiation gives no significant change to the BAP value, but has an effect of decreasing the dROM value. It was considered that the effect was brought about by the reduction action based on the Yubi-MR.

**[Table 1]**

| Human | BAP Before | dROM Before | BAP After | dROM After | BAP/dROM Before | BAP/dROM After |
|---|---|---|---|---|---|---|
| No.1 | 2223 | 364 | 2214 | 323 | 6.107 | 6.854 |
| No.2 | 2224 | 237 | 2213 | 219 | 9.383 | 10.105 |
| No.3 | 2486 | 222 | 2448 | 188 | 11.198 | 13.021 |
| No.4 | 2491 | 241 | 2416 | 231 | 10.336 | 10.458 |
| No.5 | 2537 | 266 | 2503 | 261 | 9.537 | 9.590 |
| No.6 | 2381 | 201 | 2451 | 186 | 11.845 | 13.177 |
| N.o7 | 2444 | 267 | 2404 | 224 | 9.153 | 10.732 |
| No.8 | 2377 | 193 | 2468 | 187 | 12.316 | 13.197 |
| No.9 | 2447 | 256 | 2494 | 255 | 9.558 | 9.780 |
| Average | 2401.111 | 249.666 | 2401.222 | 230.44* | 9.937 | 10.761 |

| | | | | | | |
|---|---|---|---|---|---|---|
| *: The t test gave P = 0.0037. | | | | | | |

Experiment 2: Experiment using blood of mouse
Experimenters: Naomasa Yamamoto, Norifumi Yonehara, and Yuuichi Koike
Date of experiment: Oct. 22, 2009 to Feb. 7, 2010
Animals: Mice (10-week-old, male, two) were used.
Apparatus: The Yubi-MR for animal use that is of type capable of being loaded on a pipe having a diameter of 5.6 cm and a length of 20.5 cm and can radiate an electromagnetic wave with an intensity of 0.001 dBV was used.
Method: The mouse was placed in the pipe for the Yubi-MR for animal use, and was caused to freely behave for one minute with no constraints. Thereafter, the blood was collected from the heart under ether anesthesia.

Results: Fig. 6 shows the influence of the Yubi-MR for animal use on the BAP/dROM ratio for mice. As can be seen from Fig. 6, the mice exhibited the BAP/dROM ratio ranging from 14 to 26, which was different from the BAP/dROM ratio ranging from 6 to 13 for the humans, however, it was recognized that the irradiation with the Yubi-MR for animal use caused an increase in BAP/dROM in the same manner as with the Yubi-MR irradiation onto the humans. However, the number of mice was as small as two, thus it is necessary to make a verification by increasing the number of mice.
Consideration: It has been revealed that, as with the Yubi-MR irradiation onto the index finger, the irradiation onto the entire body of the mouse with the Yubi-MR for animal use also has an effect of increasing the BAP/dROM ratio.

Experiment 3: Effect of Yubi-MR for animal use on motor activity of mouse
Experimenters: Naomasa Yamamoto, Norifumi Yonehara, and Yuuichi Koike
Date of experiment: May 26, 2009 to Feb. 7, 2010
Animals: 8 mice were used.
Apparatus: The Yubi-MR for animal use that is of type capable of being loaded on a pipe having a diameter of 5.6 cm and a length of 20.5 cm and can radiate an electromagnetic wave with an intensity of 0.001 dBV was used. A dummy apparatus was also used.
Measurement: A laboratory animal locomotor activity measuring system (manufactured by Muromachi Kikai Co., Ltd.) in which a sensor for sensing infrared radiation is mounted just above each of the cages for the eight animals, and every time a mouse makes an activity (moves), one point is added by the computer was used to accumulate the motor activity every one minute, and make totaling over 24 hours. For analysis, the values integrated up to 17 hours and 12 minutes were required.
Method: The mice were placed in a pipe loaded with a Yubi-MR for animal use (having a diameter of 5.6 cm and a length of 20.5 cm) to be left for 1 min., 10 min., or 30 min., and then taken out to be moved to the respective cages for observing the motor activity. Experiment was conducted under the conditions for time period of irradiation and that of measurement as given in Table 2. The serial experiments provided with experiment numbers were carried out over five days, and the motor activity for a given mouse was obtained from the data of locomotor activity on the repetition of five times (for the five days).

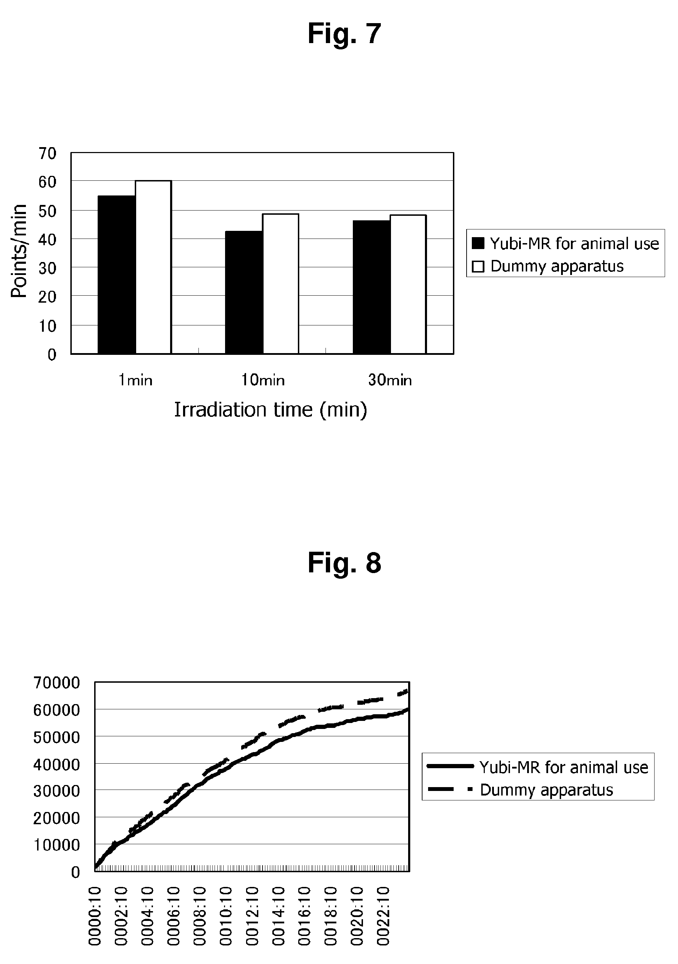

Results: The motor activity of the mice irradiated for 1 min. to 30 min. with the Yubi-MR for animal use was compared with that of the mice irradiated with the dummy apparatus. Table 3 shows the suppression of the mouse motor activity by the irradiation with the Yubi-MR for animal use and that with the dummy apparatus; Fig. 7 shows the relationship between the time period of irradiation and the motor activity for the Yubi-MR for animal use and the dummy apparatus; and Fig. 8 shows the comparison of the integrated value of the mouse motor activity on the irradiation with the Yubi-MR for animal use with that with the magnet. As shown in Table 3, it was recognized that the irradiation with the Yubi-MR for animal use caused a significantly greater reduction in motor activity than that on the irradiation with the dummy apparatus (p = 0.035). As can be seen from Fig. 7, such reduction in motor activity on the Yubi-MR for animal use was observed even when the time period of irradiation was shortened from 30 min. to 1 min. Further as shown in Fig. 8, it was found that, with the integral curve for motor activity (n = 4) up to 24 hours given when the time period of irradiation was 1 min., the influence by the Yubi-MR for animal use is not concentrated on a certain time when the mouse moves, but the reduction in motor activity is caused immediately after the irradiation with the Yubi-MR for animal use.

Consideration: It can be seen that the irradiation onto the entire body of a mouse with the Yubi-MR for animal use causes a greater reduction in motor activity than that on the irradiation with the dummy apparatus. This effect can be seen immediately after the irradiation, and could be observed even when the time period of irradiation was shortened from 30 min. to 1 min. With the dummy apparatus, such an effect can not be recognized, thus it can be considered that the effect is due to the electromagnetic wave emitted from the Yubi-MR for animal use.

**[Table 2]**

| Experiment | Number(n) of mouse | No. of Exp. | Accumulation | Irradiation |
|---|---|---|---|---|
| No.1-1 | 8 | 11x-81x | 1min | 10 min |
| No.1-2 | 8 | 91x-103x | 15 min | 30 min |
| No.2 | 8 | 104x-150x | 15 min | 30 min |
| No.3 | 8 | 160x-300x | 15 min | 30 min |
| No.4-1 | 8 | 310x-390x | 15 min | 30 min |
| No.4-2 | 8 | 400x-430x | 15 min | 10 min |
| No.5 | 8 | 450x-590x | 1 min | 10 min |
| No.6 | 8 | 600x-770x | 1 min | 1 min |
| No.7 | 8 | 800x-890x | 1 min | 1 min |
| No.8 | 8 | 900x-930x | 1 min | 1min |

**[Table 3]**

| Yubi-MR for animal use (points/min) | Dummy apparatus (points/min) |
|---|---|
| 48.7 ± 8.3 | 52.7 ± 7.7 |
| n=64 | n=64 |

Experiment 4: Recovery from effect of motor activity attenuation by Yubi-MR for animal use Experimenters: Naomasa Yamamoto, Norifumi Yonehara, and Yuuichi Koike
Date of experiment: Nov. 9, 2009 to Feb. 7, 2010
Purpose: It was studied whether the mice the motor activity of which was reduced due to the action of the Yubi-MR for animal use can restore the motor activity thereof when the irradiation is changed over to that with the magnet.
Method: The four mice having the mouse numbers No. 1 to 4 that were irradiated with the Yubi-MR for animal use for one minute, and confirmed for attenuation of the motor activity were irradiated with the dummy apparatus on the second week for one minute, while the four mice having the mouse numbers No. 5 to 8 that were irradiated with the dummy apparatus were irradiated with the Yubi-MR for animal use.
Measurement of time period of motor activity interruption: The laboratory animal locomotor activity measuring system (manufactured by Muromachi Kikai Co., Ltd.) was used to measure the mouse motor activity in units of 1 min. for monitoring over 24 hours. The case where there was no motor activity during a time period of one minute for measurement was converted into one point to calculate the 24-hour value using Excel.

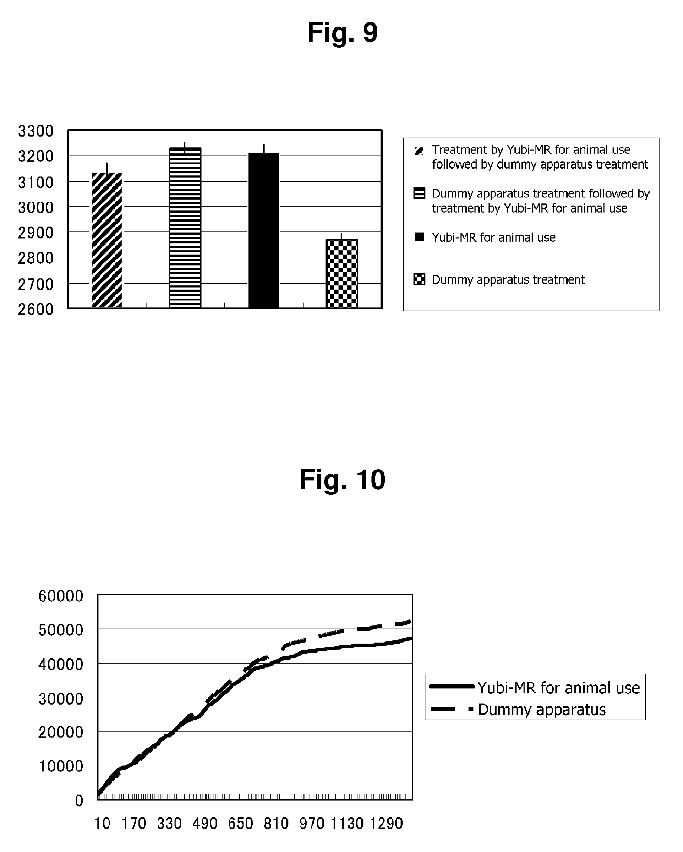

Results: As shown in Fig. 9, the mice with which the time period of motor activity interruption was extended on the irradiation with the Yubi-MR for animal use tend to restore the motor activity thereof with the irradiation being changed over to that with the magnet. The ordinate denotes the time period in minutes per 24 hours during which the mice interrupted the motor activity (the total for n = 4 and the standard error). In another experiment, Fig. 10 shows the recovery from the reduction in motor activity on the irradiation with the Yubi-MR for animal use. The ordinate denotes the motor activity (points) per one mouse, while the abscissa denotes the time period. Specifically, the mice (n = 4) with which it was confirmed that the motor activity was reduced on the irradiation with the Yubi-MR for animal use were rested for two days, and then the motor activity was further measured for five days without irradiation with the Yubi-MR for animal use. It was recognized that the mouse motor activity was increased with the interruption of the irradiation with the Yubi-MR for animal use, and it was found that the mouse motor activity was recovered in one to five days.

Consideration: This experiment was carried out in response to questions, such as how long the effect of the irradiation with the Yubi-MR for animal use is continued; whether, if the irradiation with the Yubi-MR for animal use is interrupted, the recovery of the motor activity is recognized or not; and the like. It was recognized that, when the mice were bred for two days with no treatment after having been irradiated with the Yubi-MR for animal use, and for five days from the third day, the motor activity was measured with no treatment, the motor activity was recovered. On the basis of this result, it has been considered that the interruption of the irradiation with the Yubi-MR for animal use is immediately followed by the occurrence of recovery of the motor activity.

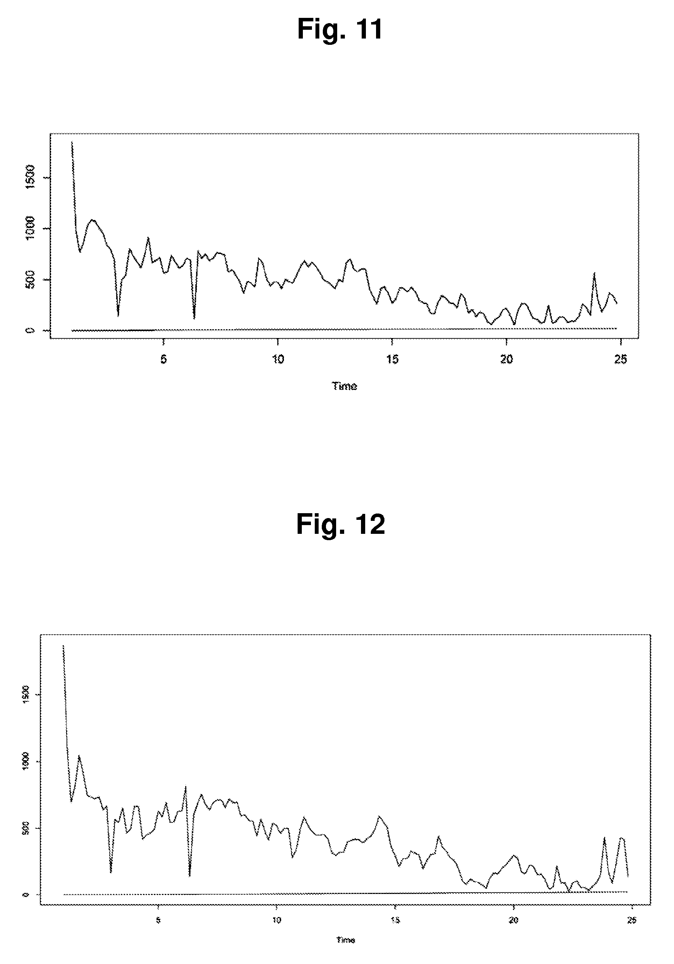

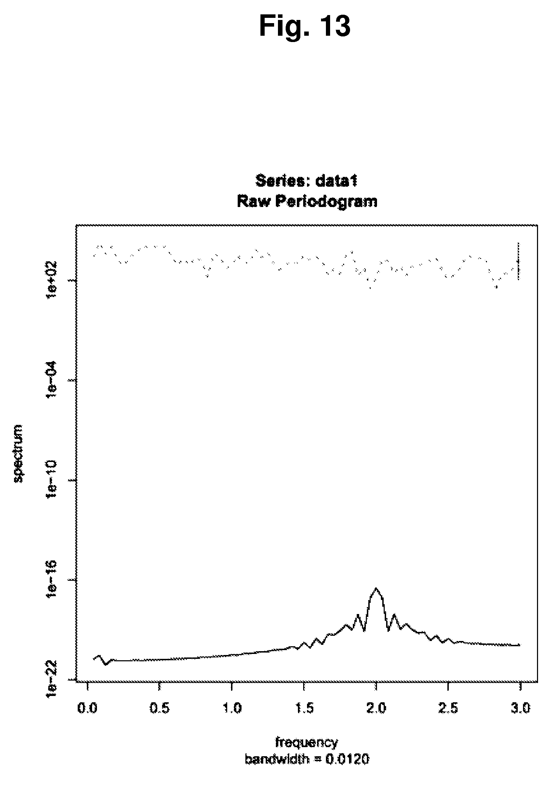

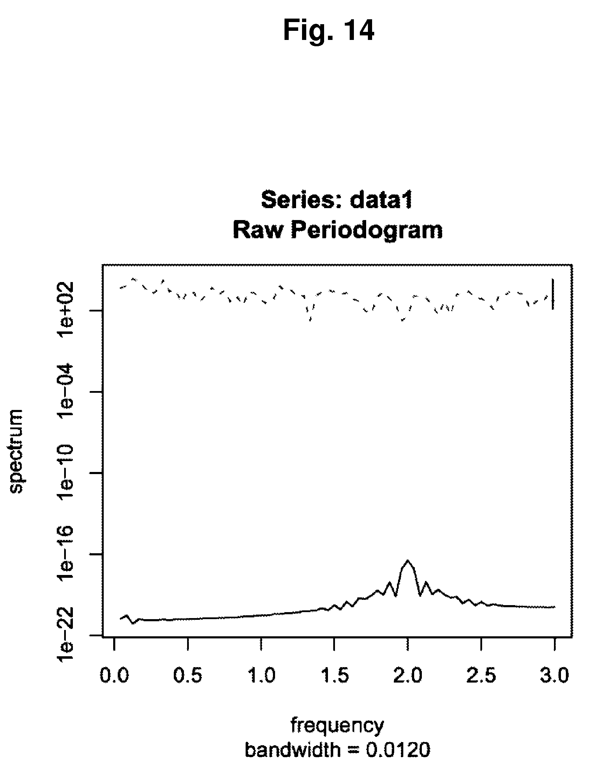

Experiment 5: Effect of Yubi-MR for animal use on motor activity rhythm
Experimenters: Naomasa Yamamoto, Norifumi Yonehara, and Yuuichi Koike
Date of experiment: May 26, 2009 to Feb. 7, 2010
Purpose: In the experiments 1 to 3, it was suggested that the Yubi-MR for animal use has an effect of suppressing the mouse motor activity. Then, it was examined whether the Yubi-MR for animal use can change the motor activity rhythm.
Method: Using the data for motor activity, the Fourier transform was performed to examine the change in frequency of the motor activity.
Results: Fig. 11 shows the frequency change in a time series on the irradiation with the magnet, while Fig. 12 shows the frequency change in a time series on the irradiation with the Yubi-MR for animal use. In Fig. 11 and Fig. 12, the unit for the abscissa is "time of day", while that for the ordinate is "points", denoting the motor activity. Fig. 13 shows the periodgram on the irradiation with the magnet, while Fig. 14 shows the periodgram on the irradiation with the Yubi-MR for animal use.
Consideration: As a result of comparing Fig. 11 with Fig. 12, and Fig. 13 with Fig. 14 for making a frequency analysis, no remarkable change in frequency was recognized. Consequently, it has been considered that the phenomenon as was seen in the present research that the mouse motor activity is reduced by the irradiation with the Yubi-MR for animal use reduces the motor activity itself without changing the pattern (frequency) of the motor activity.

Conclusion: The Yubi-MR has the ability to irradiate a special electromagnetic wave that is possessed by the Yubi-MR for animal use. The value of the ability has already been extremely highly appreciated, providing the advantages of removing the ferrous ion from the service water and preventing corrosion. The principle might be based on that the electromagnetic wave radiated from the black body acts on the water molecules, ionizing them on the NMR effect. Such a powerful effect can be expected to give electrons not only to the ferrous ions in the water, but also to the oxidized substances in the living body for making reduction. The present researchers took notice of it, and observed the influence of the Yubi-MR on the oxidative stress in the blood. As a result of it, surprisingly it was found that the irradiation onto the index finger for as short as ten minutes can significantly reduce the oxidative stress value. The same effect as this was observed also in the animal experiment, which was verified by the increase in BAP/dROM value. On the same mechanism as that which works in the case of irradiation with the Yubi-MR, the oxidized substances in the blood might have been reduced.

The Yubi-MR is a novel apparatus which utilizes a physical phenomenon, and is capable of reducing the oxidative stress in the living body. This apparatus is based on the fact that use of the electromagnetic wave (a special wave) can reduce the oxidative stress. For the purpose of examining what advantages are given to the living body by eliminating the oxidative stress, the present inventors observed the influence of doing the same on the motor activity of animals. In conducting this experiment, it is necessary to take the influence of the magnet into account. This is because, in order to amplify and filter the wave radiated from the black body, the wiggler magnet is used. It has been reported that the living beings are influenced by the magnetic field, thus the inventors tried to eliminate the influence of the magnetic field as much as possible. Before starting the animal experiment, the following problems were posed: (1) examination of the proper period of time for irradiation, (2) examination of the proper irradiation site of the animal body, and (3) setting the proper time of day when the experiment is to be started.

A dummy apparatus the magnetic flux density of which is tuned with that of the Yubi-MR for animal use was used as a control for examining the effect of the Yubi-MR for animal use. As a result of it, it was revealed that the irradiation for one minute with the Yubi-MR for animal use obviously suppressed the mouse motor activity, compared to that with the dummy apparatus. It was also revealed that such effect of the irradiation with the Yubi-MR for animal use was temporary, and when the irradiation was interrupted, the motor activity was increased. In addition, it was found that the effect of the Yubi-MR for animal use was not so great as to change the motor activity rhythm of the living beings.

We, the living creatures, have evolved over three billion and six hundred thousand years, having obtained energy by oxidizing the substance with the use of oxygen. With the earth having been changed from that with no oxygen to that with oxygen, organisms capable of utilizing an enormous amount of energy were born. The current number of living beings on the earth relates the benefit of oxygen. On the other hand, the oxidative stress can be accumulated in the body, causing senescence, accelerating arterioscelerosis, increasing the incidence of cancer, and hastening death. Therefore, it is natural to consider that, if the oxidative stress can be reduced even in some degree, it would provide a survival benefit for the living beings. From such a viewpoint, the present inventors are convinced that the Yubi-MR proposed herein is a biologically significant apparatus which provides the living beings with the reducing power to inhibit the oxidization of the blood. Hereinafter, it is expected that the safety of the Yubi-MR is enhanced, a more potent biological effect is exhibited, the research and development are advanced so as for the Yubi-MR to be popularized on the market.

### [Description of Symbols]

1: Finger insertion hole
2A, 2B, 2C, 2D: Fluid activating bodies
3: Black radiation sintered body
4: Electromagnetic wave converging body
5: Epoxy resin
10: Cover
11, 12: Straight line portion
13: Circular arc

## Claims

1. A blood oxidation inhibiting apparatus, a finger insertion hole for inserting a finger being formed therein, comprising a plurality of fluid activating bodies being disposed around the finger insertion hole, said fluid activating bodies being integrally fixed to a cover with an epoxy resin, said cover being formed of straight line portions extending substantially at right angles to each other and a circular arc portion connecting between the ends of both straight line portions in the shape of a circular arc, and said fluid activating bodies being constituted by a set of at least three fluid activating bodies, two thereof being disposed between one of both ends of the circular arc portion and said finger insertion hole and between the other of both ends of the circular arc portion and said finger insertion hole, respectively, with the electromagnetic wave converging body thereof being oriented toward said finger insertion hole, and one thereof being disposed between the corner formed by both straight line portions and said finger insertion hole with the electromagnetic wave converging body thereof being oriented toward said finger insertion hole.

2. A blood oxidation inhibiting apparatus, a finger insertion hole for inserting a finger being formed therein, comprising a plurality of fluid activating bodies being disposed around the finger insertion hole, with the electromagnetic wave converging body thereof being oriented toward said finger insertion hole, said fluid activating bodies being integrally fixed to a cover with an epoxy resin, said cover being formed substantially in the shape of a square, and said fluid activating bodies being constituted by a set of at least four fluid activating bodies each disposed between one of the four corners of said cover and said finger insertion hole with the electromagnetic wave converging body thereof being oriented toward said finger insertion hole.
